# EUROPÄISCHE PATENTSCHRIFT

(11) **EP 0 480 289 B1**
(45) Veröffentlichungstag und Bekanntmachung des Hinweises auf die Patenterteilung: **12.03.1997**
(21) Anmeldenummer: 91116745.0
(22) Anmeldetag: 01.10.1991
(51) Int. Cl.: C12Q 1/68

(54) **Verfahren zur genus- und spezies-spezifischen Detektion von Bakterien in einer Probenflüssigkeit**
Method for genus and species-specific detection of bacteria in a testliquid
Méthode de détection spécifique de genre et d'espèce de bactéries dans un liquide a prober

(30) Priorität: 09.10.1990 DE 4032024; 05.12.1990 DE 4038804
(43) Veröffentlichungstag der Anmeldung: 15.04.1992
(73) Patentinhaber: BOEHRINGER MANNHEIM GMBH, 68298 Mannheim (DE)
(72) Erfinder: Ludwig, Wolfgang, Dr. rer. nat., W-8179 Sachsenkamm (DE); Schleifer, Karl-Heinz, Prof. Dr., W-8044 Unterschleissheim (DE); Kessler, Christoph, Dr.rer.nat., W-8021 Dorfen (DE); Rüger, Rüdiger, Dr. med., W-8124 Seeshaupt (DE); Stern, Anne, Dr. rer. nat., W-8122 Penberg (DE)

(56) Entgegenhaltungen:
- EP-A- 0 307 270
- EP-A- 0 332 435
- EP-A- 0 420 260
- WO-A-90/11374
- GB-A- 2 202 328

## Beschreibung

Die Erfindung betrifft ein Verfahren zur genus- und spezies-spezifischen Detektion von Bakterien in einer Probenflüssigkeit.

Für den Nachweis bestimmter Nukleinsäuren wurden nach Entwicklung der Hybridisierungstechniken viele Methoden erarbeitet, mit deren Hilfe man das Vorhandensein oder Fehlen bestimmter Nukleinsäureabschnitte bestimmen konnte. Hierzu gehört unter anderem auch die Diagnostik von menschlichen Erbkrankheiten sowie anderen Defekten auf Genom-Ebene, die nicht unbedingt zu einer sich tatsächlich auswirkenden Krankheit führen müssen. Jedoch war die Spezifität des Nukleinsäuren-Nachweises nach den anfangs entwickelten Methoden nicht sehr hoch und es ergaben sich insbesondere beim Nachweis von Mutationen auf Nukleinsäuren, die nur wenige Basen betreffen, Schwierigkeiten hinsichtlich der Nachweisgenauigkeit. Insbesondere für die Unterscheidung von Nukleinsäure-Varianten in lediglich einer oder zwei Basen waren die bekannten Hybridisierungsmethoden zu unspezifisch. Dies lag zum einen daran, daß bei der Hybridisierung aufgrund der Verwendung relativ kurzer Oligonukleotide keine sehr stringenten Bedingungen angewandt werden konnten, weshalb meist das Fehlen der Hybridisierung einer Base (eines sogenannten "Mismatches") nicht bemerkt werden konnte. Zum anderen lag es an den oft relativ geringen verfügbaren Mengen an gereinigter nachzuweisender bzw. zu unterscheidender Nukleinsäure.

Im Rahmen der Fortentwicklung der Nukleinsäure-Nachweismethoden wurde unter anderem die PCR-Reaktion entwickelt, mit Hilfe derer eine nachzuweisende Nukleinsäure amplifiziert werden kann. Jedoch entstehen bei der PCR-Reaktion oft auch Nebenprodukte, die den nachfolgenden Nachweis durch Hybridisierung stören. Es wurde daher bereits in der EP 0 332 435 ein Verfahren vorgeschlagen, bei dem zur Unterscheidung von Nukleinsäuren, welches sich mindestens durch ein Nukleotid unterscheiden, ein diagnostischer Primer eingesetzt wird, der mit Hilfe einer Polymerase und den Nukleosidtriphosphaten elongiert wird,e ine Elongation hierbei jedoch nur dann stattfindet, wenn das 3'-endständige Nukleotid des Primers komplementär zum entsprechenden Nukleotid der nachzuweisenden Nukleinsäure ist. Die Bestimmung des Vorhandenseins einer möglichen Punktmutationsvariante wird hierbei über die An- oder Abwesenheit eines Elongationsprodukts geführt. Dies wird in dieser Anmeldung am Beispiel bestimmter Erbkrankheiten beim Menschen gezeigt.

In der Patentanmeldung GB-A-2 202 328 wird ein Verfahren zum Nachweis viraler DNS beschrieben, bei dem nachweisbar modifizierte Primer zur Target-Nukleinsäure hybridisiert und nachfolgend elongiert werden. Eine Detektion der Target-Nukleinsäure erfolgt über den Nachweis des Einbaus modifizierter Primer in den elongierten Nukleinsäurestrang.

Nun bestehen jedoch auch bei dieser Verfahrensführung Schwierigkeiten dahingehend, daß manche Polymerasen eine sogenannte "proof reading-Aktivität" besitzen, die das Mismatch bei der Primerelongation korrigieren und damit einen spezifischen Nachweis unmöglich machen. Es bestand daher nach wie vor die Notwendigkeit, ein Verfharen zu entwickeln, mit dem Unterscheide von nur einer Base auf Nukleinsäuren zuverlässig nachgewiesen werden können. Insbesondere für den Nachweis der An- oder Abwesenheit bestimmter Bakteriengattungen sowie deren Unterscheidung in einer Probenflüssigkeit bestand ein bedarf an einem hochspezifischen Nachweisverfahren für Nukleinsüäure-Varianten. Die Aufgabe der Erfindung war es daher, ein solches hochspezifisches Verfahren zur Verfügung zu stellen.

Gegenstand der Erfindung ist daher ein Verfahren zur genus- und spezies-spezifischen Detektion von Bakterien in einer Probenflüssigkeit, bei dem man bakterielle RNA mit einem Primer, der zu einer genusspezifischen Region von RNA bestimmter Bakterien komplementär ist, an seinem 3'-Ende jedoch nicht komplementär ist zur RNA von Bakterien anderer Gattung bzw. Spezies, hybridisiert, Elongation des Primers in Anwesenheit einer geeigneten Polymerase und den vier Desoxyribonukleotiden, gegebenenfalls mit gleichzeitiger oder anschließender Markierung des Elongationsproduktes bewirkt, wobei ein bei Komplementarität von Primer und Template RNA gebildetes Elongationsprodukt zusätzlich nach Denaturierung mit einem speziesspezifischen Oligonukleotid hybridisiert und die Hybridisierung über die Markierung des Oligonukleotids nachgewiesen wird.

Im Rahmen der Erfindung kann der Primer auch zu einer bei der RNA von Bakterien allgemein hochkonservierten Region komplementär sein, solange sich am 3'-Ende des Primers ein Mismatch bei der Basenpaarung mit der RNA der anderen Gattung bzw. Spezies ergibt.

Der Begriff "bakterielle RNA" umfaßt im Rahmen der Erfindung auch ein Amplifikationsprodukt der direkt von dem Bakterium gebildeten RNA. Die Amplifikation der bakteriellen RNA kann mit Hilfe bekannter Methoden, wie z.B. Polymerase chain reaction (USP 4,683,195), Nucleic acid sequence based amplification (EP-A 0 329 822) oder fast enzyme linked intense chain replication effect (deutsche Patentanmeldung P 39 29 030.1) erfolgen.

Das erfindungsgemäße Verfahren ermöglicht es, durch zwei Selektionsschritte, die Gefahr von falschen Aussagen aufgrund von nur einer unterscheidenden Reaktion deutlich zu verringern und somit ein hochspezifisches Nachweisverfahren bereitzustellen, mit dem zwischen sich auch nur in wenigen Basen unterscheidenden bakteriellen RNAs unterschieden werden kann, wodurch wiederum eine Aussage über das Vorhandensein einer bestimmten Bakteriengattung oder -spezies ermöglicht wird. Gerade die 16S-rRNA, aber auch die 23S-rRNA von Bakterien-Spezies unterscheiden sich nämlich oft nur in wenigen Nukleotiden. Solche Unterschiede stellen oft die einzige Möglichkeit dar, zwischen "schädlichen" und "harmlosen" Bakterien zu unterscheiden. Prinzipiell kann erfindungsgemäß auch eine Unterscheidung von einzelnen Bakterienfamilien erfolgen.

In einer besonders bevorzugten Ausführungsform der Erfindung wird hierbei in praktisch einem Schritt nach Untersuchung auf Anwesenheit von Vertretern einer Gattung von Bakterien gleich die Bestimmung der tatsächlichen Spezies ermöglicht. Hierzu verwendet man einen Primer, der zur RNA einer bestimmten Gattung von Bakterien komplementär ist, der jedoch an seinem 3'-Ende sich in mindestens einer, vorzugsweise zwei oder drei Basen von der RNA von Bakterien anderer Gattungen unterscheidet. Bei Anwesenheit von Bakterien der gesuchten Gattung in der Probenflüssigkeit entsteht ein Elongationsprodukt nach Primerelongation und dieses Elongationsprodukt wird dann mit einem Oligonukleotid hybridisiert, das einem anderen Bereich der Bakterien-RNA entspricht, wobei hier die Hybridisierungsvoraussetzungen nur für eine bestimmte Spezies dieser Bakterien-Gattung gegeben sind.

Im erfindungsgemäßen Verfahren ist es möglich, bei der Hybridisierung der RNA mit dem Primer sehr lange Primer zu verwenden, was es wiederum ermöglicht, sehr stringente Bedingungen bei dieser Hybridisierung anzuwenden. Hierdurch wird bereits der Primerelongationsschritt ein sehr spezifisches Resultat liefern, was durch den zusätzlichen Hybridisierungsschritt noch deutlich verbessert wird. Es ist erfindungsgemäß weiter möglich, jeweils Kontrollversuche mitlaufen zu lassen, bei denen Primer bzw. Oligonukleotide eingesetzt werden, die zu Bereichen auf der RNA komplementär bzw. identisch sind, welche für eine Vielzahl von Bakteriengattungen und -spezies gleich sind. Es existieren hier für Kontrollversuche sogar Primer bzw. Oligonukleotide, die für alle Eubakterien spezifisch sind. Durch solche Kontrollversuche kann das Vorhandensein und die Zugänglichkeit der Target-RNA gezeigt werden und somit "falsch-negative" Resultate verhindert werden. Weiter können Blindversuche ohne Target-RNA durchgeführt werden. Hierdurch wird vermieden, daß Artefakte bei sowohl der Primerelongation als auch bei der Hybridisierung mit der tatsächlichen spezifischen Reaktion verwechselt werden. Gleichzeitig kann die Menge an Bakterien bestimmter Gattungen bzw. Spezies im Vergleich zur Gesamtbakterienmenge abgeschätzt werden.

Als RNA von bakteriellem Ursprung kann erfindungsgemäß jegliche RNA eingesetzt werden, für die Unterschiede bei verschiedenen Gattungen bzw. Spezies gezeigt werden können. Vorzugsweise wird rRNA, mRNA oder t-RNA benutzt, indem hierzu komplementäre Primer und Oligonukleotide für das erfindungsgemäße Verfahren ausgewählt werden. Bakterielle RNA wird für das erfindungsgemäße Verfahren nach an sich bekannten Methoden präpariert.

Der tatsächliche Nachweis der Hybridisierung des gebildeten Elongationsprodukts erfolgt über die Markierung des Oligonukleotids, wobei hier jegliche Markierungsarten für Nukleinsäuren anwendbar sind. Auch das Elongationsprodukt kann erfindungsgemäß bereits bei der Erzeugung, jedoch auch anschließend oder über den Primer markiert werden, um auch hier die Beobachtung der Reaktion zu ermöglichen. Vorzugsweise erfolgt die Markierung des Elongationsprodukts, indem ein bereits markierter Primer eingesetzt wird.

Erfindungsgemäß ist es besonders bevorzugt, daß die Markierung des Primers und des Oligonukleotids unterschiedlich sind und daher beide Verfahrensschritte separat kontrolliert und überwacht werden können.

Die Elongationsprodukte werden zur Hybridisierung auf an sich bekannte Weise präpariert, vorzugsweise indem eine Abtrennung von nicht-elongiertem Primer und nicht-umgesetzten Nukleinsäurebausteinen erfolgt. Es ist hierbei jegliche Methode geeignet, die zu einer Größenaufspaltung von Nukleinsäurestücken führt. Vorzugsweise wird dies mit Hilfe von Gelelektrophorese oder aufsteigender Dünnschichtchromatographie bewirkt. Auch Auftrennung über HPLC erscheint geeignet.

Die Hybridisierung mit dem Oligonukleotid erfolgt dann ebenfalls in an sich bekannter Weise, wie z.B. nach Southern Transfer der DNA auf ein Nitrocellulosepapier oder andere bekannte Methoden. Aufgrund sowohl der Lokalisation des Signals nach Hybridisierung mit dem Oligonukleotid, als auch durch die Signalstärke können dann die nötigen Rückschlüsse gezogen werden.

Es ist jedoch im Rahmen der Erfindung auch möglich und ebenfalls bevorzugt, eine Doppelmarkierung von Elongationsprodukt und Oligonukleotid in einer solchen Weise durchzuführen, daß ein gebildetes Elongationsprodukt über seine Markierung an eine feste Phase gebunden wird und dann der Nachweis über ein Oligonukleotid in markierter Form geführt wird, das zum nichtverlängerten Primer nicht komplementär ist und dadurch auch kein falsches Signal geben kann. Für solche Markierungen sind z.B. Bindungspaare wie Biotin/Streptavidin oder Hapten/ Anti-Hapten-Anitkörper, wie Digoxigenin/Anti-Digoxigenin-Antikörper, geeignet, bei denen ein Bindungspartner an die feste Phase gekoppelt wird (z.B. Streptavidin) und der zweite Partner an den Primer oder einen der Nukleinsäurebausteine gebunden wird.

Um eben die Spezifität des erfindungsgemäßen Verfahrens zu erhöhen, findet die Hybridisierung mit dem Oligonukleotid an eine Sequenz statt, die von der Primer-Sequenz verschieden ist. Diese Sequenz kann in einer Entfernung von der Primersequenz liegen, wie üblicherweise Primerelongationsverfahren entsprechende Nukleinsäurekettenlängen liefern. Vorzugsweise findet die Hybridisierung mit einer Sequenz statt, die 20 bis 400 Basen in 5'-Richtung benachbart zur zum Primer komplementären Sequenz auf der RNA liegt.

Im Rahmen des erfindungsgemäßen Verfahrens wird vorzugsweise ein Primer eingesetzt, der 15 bis 50 Nukleotide lang ist, wobei jedoch die Länge des Primers primär von den Hybridisierungsbedingungen und damit der Stringenz des Verfahrens abhängt, was wiederum von der Temperatursensitivität der verwendeten Polymerase, nämlich vorzugsweise AMV- oder MMuLV-Reverse Transkriptase, abhängig ist. Die Hybridisierungstemperatur darf nicht höher sein als die Temperaturtoleranz der Polymerase zuläßt, sollte jedoch hoch genug sein, um eine hohe Stringenz zu ermöglichen. Je länger der Primer ist, um so höher ist die Schmelztemperatur des Hybridproduktes aus Primer und nachzuweisender RNA-Sequenz.

Das zur Hybridisierung mit dem Elongationsprodukt verwendete Oligonukleotid ist im Rahmen der Erfindung bevorzugt 12 bis 20 Nukleotide lang, wodurch auf der einen Seite eine hohe Spezifität erreicht wird, auf der anderen Seite jedoch die Anforderungen an die Oligonukleotidsynthese relativ gering gehalten werden.

Das erfindungsgemäße Verfahren ermöglicht es, in hochspezifischer Weise in einer Probenflüssigkeit die Anwesenheit bestimmter Bakterien nachzuweisen. Es wird hierbei insbesondere ermöglicht, bei Anwesenheit einer bestimmten Bakteriengattung gleich auch die tatsächliche Subspezies zu bestimmen. Gleichzeitig wird den hohen Spezifitätsanforderungen der klinischen Diagnostik genügt.

Die folgenden Beispiele sollen die Erfindung weiter erläutern.

### Beispiel 1

### Differenzierung von Lactococcus lactis subspecius lactis und L. lactis subspec. cremoris

Zellen der jeweiligen Bakteriensubspezies (1 bis 3 g) wurden in 3 ml 1 x SSC (150 mmol/l NaCl, 15 mmol/l Trinatriumcitrat, pH 7,0 bei 0°C) suspendiert. Nach Zellaufschluß mit Glasper(50 g; φ 0,17 mm) im Zellhomogenisator für 4 x 30 Sekunden wurde eine Phenolextraktion durchgeführt, bei der 3 x mit je 3 ml 1 x SSC und 6 ml Phenol (1 x SSC gesättigt) ausgeschüttelt wurde. Darauf folgte eine viermalige Extraktion mit dem doppelten Volumen an Diethylether. Schließlich wurde eine Fällung der Nukleinsäuren durch Zugabe von 0,1 Vol. 5 mol/l Kaliumacetat, 2,5 Vol. abs. Ethanol bei -20°C durchgeführt und nach Zentrifugation (20 Minuten, 10.000 g) der Niederschlag im Vakuum getrocknet. Vorhandene DNA wurde durch Suspendieren in 10 ml 3 mol/l Natriumacetat, pH 6,0 bei 0°C im Ultra-Turrax (10 Sekunden) resuspendiert und nach Zentrifugation und Wiederholung dieser Prozedur über 3 bis 4 x als Pellet vornehmlich die rRNA erhalten.

2 µg der so hergestellten rRNA und 10 pmol Primer der folgenden Sequenz (SEQ ID NO 1) 5′ CGGTTAAGAACGCTCTCCTACCATTT 3′ wurden in 5 ml Puffer (0,1 mol/l KCl; 0,05 mol/l Tris-HCl, pH 8,3) 4 Minuten bei 90°C denaturiert und das Gemisch danach 10 Minuten bei 70°C inkubiert. Hiervon wurde 1 µl mit 4 µl Puffer (0,05 mol/l Tris/HCl, pH 8,3; 0,12 mol/l NaCl, 0,07 mol/l MgCl₂; je 0,06 mmol/l dGTP, dCTP, dTTP; 0,06 mmol/l [³²P] α-dATP (ungefähr 1 µCi; 5 Einheiten AMV oder MMuLV-Reverse Transkriptase (Boehringer Mannheim)) 20 Minuten bei 45°C inkubiert. Die Auftrennung der Reaktionsprodukte (2 µl) erfolgte mittels aufsteigender Dünnschichtchromatographie (DEAE-Cellulose, Polygram cell 300, Schleicher und Schüll) bei 80°C über 45 Minuten mit dem Laufmittel 0,4 mol/l Ammoniumformiat in 9 mol/l Harnstoff. Nach Autoradiographie konnten die gebildeten Elongationsprodukte gezeigt werden.

Als Kontrolle des Nachweissystems wurde in analoger Weise eine Kontrollreaktion als Positivstandard mit Hilfe eines synthetischen Oligonukleotids, das komplementär zu rRNA-Molekülen von praktisch allen Bakterien ist, mit der Sequenz (SEQ ID NO 5′ AGAACGCTCCCCTACC 3′ durchgeführt. Die Bedingungen waren wie bereits für den spezifischen Primer ausgeführt.
Eine Probe der Elongationsprodukte, welche mit Hilfe des spezifischen Primers erhalten wurden, wurde in 200 µl 0,4 N NaOH denaturiert (20 Minuten, 25°C) und dann an eine Membran (Zetaprobe, Biorad) gebunden (dot blot, Minifold, Schleicher und Schüll). Die Detektorsonde mit der Sequenz (SEQ ID NO 3) 5′ TGCGTAATAGCTCACT 3′, deren komplementäre Sequenz auf der RNA ca. 65 Basen 5' der zum spezifischen Primer komplementären Sequenz liegt (5 pmol) wurde in 20 µl Puffer (0,027 mol/l Tris/HCl, pH 8; 0,011 mol/l DTT; 0,9 mmol/l Spermin; 0,011 mol/l MgCl₂; 0,25 mmol/l [γ-³²P] ATP, ca. 30 µCi) markiert durch 30-minütige Inkubation bei 37°C. Durch Anwendung des Systems Gene Clean™ oder Mer Maid (Bio 101 Inc., La Jolla, USA) wurden nicht umgesetzte Nukleotide abgetrennt und die markierte Detektorsonde gereinigt. Die wie oben vorbereitete Membran, an die das Elongationsprodukt gebunden ist, wurde in 0,5 x SSC, 0,5 % SDS eine Stunde bei 50°C gewaschen und dann in 6 x SSC, 5 x Denhardt, 0,5 % Sarkosyl, 0,1 % SDS 2 Stunden ebenfalls bei 50°C vorinkubiert. Die Hybridisierung erfolgte mit 3 pmol der Detektorsonde in 6 x SSC, 5 x Denhardt, 0,5 % Sarcosyl, 0,1 % SDS über 4 Stunden bei 38°C, worauf mit 2 x SSC, 0,1 % SDS (2 x 5 Minuten 25°C, 1 x 5 Minuten 37°C) gewaschen wurde. Nach Autoradiographie wurde eine densitometrische Analyse des Autoradiogramms durchgeführt, deren Ergebnis zeigt, daß nach Aufbringen der Elongationsprodukte im Dot blot vergleichbare Signale bei den gebildeten Elongationsprodukten und der Kontrollreaktion erhalten wurden, bei Verwendung der Detektorsonde jedoch nur bei Anwesenheit von L. lactis subspec. lactis.

### Beispiel 2

### Differenzierung von Lactococcus lactis und Streptococcus oralis, Nachweis von cDNA mit L. lactis-spezifischer Detektorsonde

Als Primer wurde das 17 Basen lange Oligonukleotid mit der Sequenz (SEQ ID NO 4) 5′ TTCGCTCGCCGCTACTT 3′ eingesetzt, das zu RNA von L. lactis komplementär ist, sich von Streptococcus oralis-RNA jedoch in der letzten Base an seinem 3'-Ende unterscheidet. Die RNA-Präparation sowie die Hybridisierung und Primerelongation erfolgten wie in Beispiel 1. Als spezifisches, nur an L. lactis-RNA hybridisierendes Oligonukleotid wurde die Sequenz (SEQ ID NO 5) 5′ CTAGCAGTTATTCATGAGTG 3′ eingesetzt, die erhebliche Unterschiede zur RNA-Sequenz von S. oralis aufweist. Die Hybridisierung des auf Membran übertragenen Elongationsprodukts erfolgte ebenfalls wie in Beispiel 1 beschrieben, jedoch bei einer Temperatur von 48°C. Der Nachweis erfolgte wie in Beispiel 1 durch Autoradiographie und densitometrische Auswertung.

### Beispiel 3

### Differenzierung von Lactococcus-Gattungen von anderen Enterokokken oder grampositiven Bakterien und Subspezies-spezifische Unterscheidung von Lactococcus lactis Subspezies lactis und Lactococcus lactis Subspezies cremoris

Als Primer wurde die Sequenz (SEQ ID NO 6) 5′ CTCACTTCTTAACGCTCCAG 3′ verwendet. Diese Sequenz ist gattungsspezifisch für Lactokokken und ermöglicht die Unterscheidung dieser Gattung von Enterokokken und anderen grampositiven Bakterien. Die RNA-Präparation sowie die Hybridisierung und Primer-Elongation erfolgte wie in Beispiel 1. Als spezifisch für die Subspezies Lactococcus lactis Subspezies lactis spezifisches Oligonukleotid wurde die Sequenz (SEQ ID NO 7) 5' AGCGTTGGATTCAATTTAAT 3' verwendet. Die Hybridisierung des auf Membran übertragenen Elongationsproduktes erfolgte wiederum bei 38°C und auch im übrigen wie in Beispiel 1 beschrieben. Nach Autoradiographie und densitometrischer Auswertung ergab sich hier eindeutig eine Hybridisierung nur bei Anwesenheit von Lactococcus lactis Subspezies lactis, nicht jedoch bei Anwesenheit von lediglich Elongationsprodukten von Lactococcus lactis Subspezies cremoris.

### Sequenzprotokoll

### SEQ ID NO 1

| | |
|---|---|
| Sequenzlänge: | 26 Basen |
| Art der Sequenz: | Nukleotidsequenz (DNA) |
| Strangform: | Einzelstrang |
| Topologie: | Linear |

5'-CGGTTAAGAA CGCTCTCCTA CCATTT-3'

### SEQ ID NO 2

| | |
|---|---|
| Sequenzlänge: | 16 Basen |
| Art der Sequenz: | Nukleotidsequenz (DNA) |
| Strangform: | Einzelstrang |
| Topologie: | Linear |

5'-AGAACGCTCC CCTACC-3'

### SEQ ID NO 3

| | |
|---|---|
| Sequenzlänge: | 16 Basen |
| Art der Sequenz: | Nukleotidsequenz (DNA) |
| Strangform: | Einzelstrang |
| Topologie: | Linear |

5'-TGCGTAATAG CTCACT-3'

### SEQ ID NO 4

| | |
|---|---|
| Sequenzlänge: | 17 Basen |
| Art der Sequnez: | Nukleotidsequenz (DNA) |
| Strangform: | Einzelstrang |
| Topologie: | Linear |

5'-TTCGCTCGCC GCTACTT-3'

### SEQ ID NO 5

| | |
|---|---|
| Sequenzlänge: | 20 Basen |
| Art der Sequenz: | Nukleotidsequenz (DNA) |
| Strangform: | Einzelstrang |
| Topologie: | Linear |

5'-CTAGCAGTTA TTCATGAGTG-3'

### SEQ ID NO 6

| | |
|---|---|
| Sequenzlänge: | 20 Basen |
| Art der Sequenz: | Nukleotidsequenz (DNA) |
| Strangform: | Einzelstrang |
| Topologie: | Linear |

5'-CTCACTTCTT AACGCTCCAG-3'

### SEQ ID NO 7

| | |
|---|---|
| Sequenzlänge: | 20 Basen |
| Art der Sequenz: | Nukleotidsequenz (DNA) |
| Strangform: | Einzelstrang |
| Topologie: | Linear |

5'-AGCGTTGGAT TCAATTTAAT-3'

## Patentansprüche

1. Verfahren zur genus- und spezies-spezifischen Detektion von Bakterien in einer Probenflüssigkeit, mit den Schritten
- bakterielle RNA wird mit einem Primer, der zu einer genus-spezifischen Region von RNA bestimmter Bakterien oder einer hochkonservierten Region der RNA von Bakterien allgemein komplementär ist, an seinem 3'-Ende jedoch nicht komplementär ist zur RNA von Bakterien anderer Gattung hybridisiert,
- Elongation des Primers in Anwesenheit einer geeigneten Polymerase und den vier Desoxyribonukleotiden, gegebenenfalls mit gleichzeitiger oder anschließender Markierung des Elongationsproduktes,
- ein gebildetes Elongationsprodukt wird nach Denaturierung mit einem spezies-spezifischen Oligonukleotid hybridisiert,
- die Hybridisierung wird über die Markierung des Oligonukleotids nachgewiesen.

2. Verfahren nach Anspruch 1, dadurch gekennzeichnet, daß man Primer und Oligonukleotide verwendet, die zu einer genus- bzw. spezies-spezifischen Region von rRNA, mRNA oder tRNA komplementär bzw. identisch sind.

3. Verfahren nach einem der Ansprüche 1 oder 2, dadurch gekennzeichnet, daß man unterschiedliche Markierungen für Primer und Oligonukleotid verwendet.

4. Verfahren nach einem der vorangehenden Ansprüche, dadurch gekennzeichnet, daß man als Polymerase AMV- oder MMuLV-Reverse Transkriptase verwendet.

5. Verfahren nach einem der vorhergehenden Ansprüche, dadurch gekennzeichnet, daß man die gebildeten Elongationsprodukte vor der Hybridisierung mit dem Oligonukleotid mit Hilfe von Gelelektrophorese oder aufsteigender Dünnschichtchromatographie größenabhängig auftrennt.

6. Verfahren nach einem der Ansprüche 1 bis 4, dadurch gekennzeichnet, daß man als Markierung für das Elongationsprodukt den einen Partner eines spezifischen Bindepaares verwendet, dessen anderer Partner an eine feste Phase gebunden ist und die Hybridisierung mit dem Oligonukleotid an dieser festen Phase erfolgt.

7. Verfahren nach einem der vorhergehenden Ansprüche, dadurch gekennzeichnet, daß die Hybridisierung mit dem Oligonukleotid an einer Sequenz stattfindet, die 20 bis 400 Basen in 5'-Richtung benachbart zur zum Primer komplementären Sequenz liegt.

8. Verfahren nach einem der vorhergehenden Ansprüche, dadurch gekennzeichnet, daß das Oligonukleotid 12 bis 20 Nukleotide lang ist.

9. Verfahren nach einem der vorhergehenden Ansprüche, dadurch gekennzeichnet, daß der Primer 15 bis 50 Nukleotide lang ist.

## Claims

1. Method for the genus-specific and species-specific detection of bacteria in a sample liquid comprising the steps
- bacterial RNA is hybridized with a primer which is complementary to a genus-specific region of the RNA of particular bacteria or to a highly conserved region of the RNA of bacteria in general, but which is not complementary at its 3' end to the RNA of bacteria of another genus,
- the primer is elongated in the presence of a suitable polymerase and the four deoxyribonucleotides, if desired, with a concurrent or subsequent labelling of the elongation product,
- an elongation product formed is hybridized with a species specific oligonucleotide after denaturation,
- the hybridization is detected by means of the oligonucleotide label.

2. Method as claimed in claim 1, wherein primers and oligonucleotides are used which are complementary or identical to a genus-specific or species-specific region of rRNA, mRNA or t-RNA.

3. Method as claimed in one of the claims 1 or 2, wherein different labels are used for the primer and the oligonucleotide.

4. Method as claimed in one of the previous claims, wherein AMV- or MMuLV-reverse transcriptase is used as the polymerase.

5. Method as claimed in one of the previous claims, wherein the elongation products formed are separated according to their size using gel electrophoresis or ascending thin layer chromatography before hybridization with the oligonucleotide.

6. Method as claimed in one of the claims 1 to 4, wherein one partner of a specific binding pair is used as the label for the elongation product and its other partner is bound to a solid phase and the hybridization with the oligonucleotide is carried out on this solid phase.

7. Method as claimed in one of the previous claims, wherein the hybridization with the oligonucleotide takes place on a sequence neighbouring the sequence which is complementary to the primer and is at a distance of 20 to 400 bases in the 5' direction.

8. Method as claimed in one of the previous claims, wherein the oligonucleotide is 12 to 20 nucleotides long.

9. Method as claimed in one of the previous claims, wherein the primer is 15 to 50 nucleotides long.

## Revendications

1. Procédé de détection, spécifique du genre et de l'espèce, de bactéries dans un échantillon liquide, comprenant les étapes consistant
- à hybrider de l'ARN bactérien avec une amorce essentiellement complémentaire d'une région spécifique du genre de l'ARN de bactéries déterminées ou d'une région bien conservée de l'ARN de bactéries, l'amorce n'étant toutefois pas complémentaire, à son extrémité 3', de l'ARN de bactéries d'un autre genre,
- à allonger l'amorce en présence d'une polymérase appropriée et de quatre désoxyribonucléotides, éventuellement avec marquage simultané ou subséquent du produit de l'allongement,
- à hybrider un produit d'allongement formé, après dénaturation, avec un oligonucléotide spécifique de l'espèce,
à détecter l'hybridation par l'intermédiaire du marqueur de l'oligonucléotide.

2. Procédé selon la revendication 1, caractérisé en ce que l'on utilise une amorce et un oligonucléotide qui sont complémentaires de, ou identiques à une région, spécifique du genre ou de l'espèce, d'ARNr, d'ARNm ou d'ARNt.

3. Procédé selon l'une des revendications 1 ou 2, caractérisé en ce que l'on utilise des marqueurs différents pour l'amorce et pour l'oligonucléotide.

4. Procédé selon l'une quelconque des revendications précédentes, caractérisé en ce que l'on utilise, en tant que polymérase, la transcriptase inverse AMV ou MMuLV.

5. Procédé selon l'une quelconque des revendications précédentes, caractérisé en ce qu'avant l'hybridation avec l'oligonucléotide, on sépare les produits d'allongement formés, en fonction de leur taille, au moyen d'une électrophorèse sur gel ou d'une chromatographie en couche mince ascendante.

6. Procédé selon l'une quelconque des revendications 1 à 4, caractérisé en ce que, comme marqueur du produit d'allongement, on utilise l'un des partenaires d'un couple de liaison spécifique dont l'autre partenaire est lié à une phase solide et l'hybridation avec l'oligonucléotide s'effectue sur cette phase solide.

7. Procédé selon l'une quelconque des revendications précédentes, caractérisé en ce que l'hybridation avec un oligonucléotide se produit au niveau d'une séquence qui est située à une distance de 20 à 400 bases, en direction 5', de la séquence complémentaire de l'amorce.

8. Procédé selon l'une quelconque des revendications précédentes, caractérisé en ce que la longueur de l'oligonucléotide est de 12 à 20 nucléotides.

9. Procédé selon l'une quelconque des revendications précédentes, caractérisé en ce que la longueur de l'amorce est de 15 à 50 nucléotides.
